# FASCICULE DE BREVET EUROPEEN

(11) **EP 3 019 590 B1**
(45) Date de publication et mention de la délivrance du brevet: **23.08.2017**
(21) Numéro de dépôt: 14739774.9
(22) Date de dépôt: 10.07.2014
(51) Int. Cl.: C12M 3/06, C12M 1/12

(54) **NOUVEAU PROCÉDÉ POUR LA CULTURE DE MICROORGANISMES PAR CONFINEMENT DANS DES MICRO-BIORÉACTEURS**
NEUARTIGES VERFAHREN ZUR KULTIVIERUNG VON MIKROORGANISMEN DURCH HALTUNG IN MIKROBIOREAKTOREN
NOVEL METHOD FOR CULTIVATING MICRO-ORGANISMS BY CONFINEMENT IN MICRO-BIOREACTORS

(30) Priorité: 10.07.2013 FR 1301631
(43) Date de publication de la demande: 18.05.2016
(73) Titulaire: Etablissements J. Soufflet, 10400 Nogent Sur Seine (FR); Ecole Supérieure de Physique et de Chimie Industrielles de la Ville de Paris, 75005 Paris (FR)
(72) Inventeur: GARNICA RODRIGUEZ, Jairo, Ivan, 91300 Massy (FR); BOITARD, Laurent, 75010 Paris (FR); DREVELLE, Antoine, Serge, Dominique, F-10100 Romilly (FR); BIBETTE, Jérôme, F-75005 Paris (FR)
(74) Mandataire: Regimbeau
(86) Numéro de dépôt international: PCT/EP2014/064800
(87) Numéro de publication internationale: WO 2015/004228

(56) Documents cités:
- FR-A1- 2 972 198
- US-B1- 6 458 335

## Description

La présente invention concerne un nouveau procédé pour la culture de microorganismes par confinement dans des micro-bioréacteurs.

De manière plus précise, la présente invention permet de suivre de manière cinétique et sur de longs temps d'incubation (>24 h) la culture de microorganismes en milieu confiné.

Il est connu, notamment par la demande de brevet français n° 11/00659, de procéder à la culture en milieu confiné de microorganismes ; cette demande décrit notamment un procédé et un dispositif de suivi de réaction en cinétique avec un confinement des microorganismes dans des réservoirs aqueux. Ce procédé repose sur le référencement de la séquence des réservoirs aqueux au sein d'une succession de réservoirs aqueux séparés par un fluide d'espacement et transporté par un fluide porteur dans un tube. Cependant ledit procédé ne permet pas d'étudier des microorganismes dont la croissance ou l'activité biologique va modifier les interfaces. En effet, dans ces cas, au-delà de 24h, l'intégrité des réservoirs n'est plus préservée : soit ils fusionnent entre eux, soit ils se subdivisent en réservoirs plus petits. L'interprétation des résultats de l'expérience n'est plus possible dès lors qu'au moins un des réservoirs a été perdu, le référencement étant perdu également et/ou lorsque la taille de l'un desdits réservoirs varie, par exemple parce qu'il fuit ; dans ce dernier cas, la concentration en métabolite que l'on mesure à l'intérieur va être artificiellement augmentée par effet de concentration et non parce que le microorganisme contenu dans ledit réservoir est superproducteur du métabolite en question.

En règle générale, les technologies existantes ne permettent pas de confiner efficacement tous les types de microorganismes sur des durées supérieures à 24h.

L'objet du procédé selon l'invention est de pouvoir étudier un cycle de vie entier quels que soient le type de microorganismes, leurs activités biologiques et leurs vitesses de croissance.

De façon plus précise, le procédé selon l'invention pour la culture de microorganismes par confinement dans des micro-bioréacteurs, est du type comprenant un tube capillaire dans lequel circule un fluide porteur destiné à faire avancer un train de gouttes constitué de micro-bioréacteurs dans lesquels a lieu la culture desdits microorganismes, où lesdites micro-bioréacteurs sont séparés par un fluide d'espacement, ce dernier étant un gaz.

Selon un mode préférentiel de l'invention, le diamètre des micro-bioréacteurs dans lesquels a lieu la culture desdits microorganismes est inférieur à celui dudit tube capillaire.

Avantageusement, la taille de la bulle dudit fluide d'espacement est comprise dans une fourchette de deux à dix fois le diamètre dudit tube capillaire.

Le procédé selon la présente invention sera mieux compris à la lecture de la description qui va suivre, faite en regard des figures données à titre purement indicatif, parmi lesquelles :
- la figure 1 représente de manière schématique la constitution du train de gouttes selon l'état de la technique ;
- la figure 2 est une vue en coupe longitudinale d'un capillaire à l'intérieur duquel circule un train de gouttes selon l'état de la technique ;
- la figure 3 est une vue en coupe longitudinale d'un capillaire à l'intérieur duquel circule un train de gouttes selon l'invention ; et
- la figure 4 représente de façon simplifiée l'ensemble du dispositif de mise en oeuvre du procédé selon l'invention.

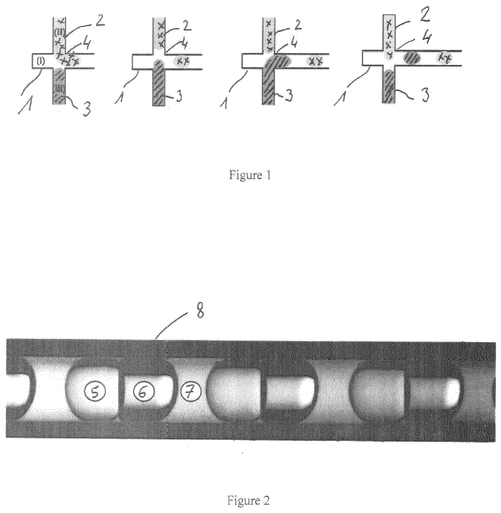

Ainsi que le représente la figure 1, le train de gouttes est constitué de façon habituelle à partir de trois phases (I), (II) et (III), non miscibles entre elles, chaque phase provenant d'un réservoir non représenté ici, des vannes (par exemple des électrovannes ou des vannes pneumatiques), non illustrées, permettant la libération des différentes phases dans leur tube respectif (1), (2) et (3) convergeant vers une jonction en croix, où se forment lesdites gouttes et dont la branche (4) est le tube capillaire dans lequel circule le train de gouttes. La phase (I) constitue le fluide porteur, la phase (II) constitue les gouttes dans lesquelles les microorganismes sont cultivés et la phase (III) constitue le fluide d'espacement ainsi qu'il sera précisé plus avant dans la présente description. La taille et l'espacement entre les différentes gouttes dépendent de la géométrie de la jonction et du ratio des débits d'injection des phases. Enfin, l'encapsulation de microorganismes à l'intérieur des gouttes de la phase (II) suit une loi de Poisson.

L'art antérieur, notamment la demande de brevet français n° 11/00659, enseigne comment se constitue le train de gouttes précédemment décrit; la présente invention ne concerne pas ce procédé d'obtention d'un tel train de gouttes.

La figure 2 permet de mieux voir une partie du train de gouttes traditionnel constitué de gouttes (5) contenant le mélange réactionnel dans lequel le microorganisme va se développer, séparées les unes des autres par des gouttes de fluide d'espacement (6) empêchant les gouttes (5) de fusionner ; les gouttes (5) et (6) sont poussées par un fluide porteur (7) à l'intérieur d'un tube capillaire (8), ledit fluide porteur (7) permettant à la fois de déplacer les gouttes et de lubrifier le capillaire (8), évitant la contamination entre gouttes (5) consécutives. On entend par tube capillaire un tube dont le diamètre interne est inférieur à 2mm.

Comme cela a été précédemment mentionné, le train de gouttes est constitué de trois phases non-miscibles. Le fluide porteur (I) est le plus souvent une huile perfluorée («téflon liquide ») qui ne présente aucune toxicité pour le microorganisme contenu dans le micro-bioréacteur. Afin d'assurer la bonne formation du train et d'éviter tout problème de contamination entre les gouttes, l'huile porteuse a une affinité pour le capillaire supérieure aux autres phases.

La deuxième phase (II) aqueuse contient les cellules et le milieu de culture.

Enfin, la troisième phase (III) ne se mélange pas avec les deux premières ; elle peut consister en un liquide tel qu'un hydrocarbure ou une huile minérale.

L'état de la technique concernant la formation d'un train de gouttes permettant la culture de microorganismes dans ces gouttes ayant été rappelé, la présente description va maintenant aborder les améliorations apportées aux procédés connus, palliant ainsi les inconvénients desdits procédés.

Dans la suite de la présente description, les gouttes (5) dans lesquelles sont cultivés les microorganismes seront dénommées les micro-bioréacteurs (5).

Le procédé selon la présente invention est applicable à différents microorganismes et notamment aux champignons filamenteux et aux algues planctoniques.

Les champignons filamenteux forment des filaments hydrophobes (hyphes) pouvant s'étendre d'un micro-bioréacteur à un autre à travers le liquide porteur. Dans le cas où on utilise un liquide d'espacement composé d'un hydrocarbure, les filaments vont pouvoir traverser complètement jusqu'au micro-bioréacteur voisin. Ils peuvent aussi former des biofilms à l'interface micro-bioréacteur/hydrocarbure qui vont peu à peu obstruer totalement la section du capillaire. Ce phénomène aboutit à la destruction du train et à l'arrêt de l'expérience.

Afin de résoudre ce problème, il a été trouvé, et c'est là l'une des caractéristiques du procédé selon l'invention, qu'il était possible d'utiliser comme fluide d'espacement un gaz. Toutefois, si le micro-bioréacteur est trop grand et qu'il a une surface de contact importante avec le capillaire, le phénomène d'obstruction dû au développement du champignon filamenteux peut également se produire. Se posent ainsi des problèmes de contamination entre les micro-bioréacteurs (5) ainsi que de stabilité du train de gouttes dû aux interactions des filaments avec le capillaire (8).

Dans le cas des algues planctoniques, il a été observé sur les bords du train de gouttes un phénomène d'auto-émulsification dans les micro-bioréacteurs aqueux (5) ainsi que dans les bulles d'espacement (6). Cela signifie que spontanément des gouttelettes d'une phase dans l'autre se forment dans les deux types de compartiments. L'explication la plus probable est la présence de bactéries qui cohabitent avec les algues dans les micro-bioréacteurs. Ces bactéries sont capables de synthétiser des surfactants qui vont favoriser l'auto-émulsification et ainsi entraîner l'effondrement du train de gouttes.

S'agissant des algues planctoniques, utiliser un gaz comme fluide d'espacement suffit à résoudre le problème d'auto-émulsification.

Toutefois dans le cas des champignons filamenteux, ce remplacement ne suffit pas à assurer la stabilité du train. La solution consiste alors, et c'est là une autre caractéristique du procédé selon l'invention, à réduire la taille du micro-bioréacteur (5) afin de diminuer les interactions des filaments avec les parois du tube capillaire (8).

Parmi les différents gaz servant de fluide d'espacement, on utilisera avantageusement l'air, d'une part en raison de ses propriétés de mouillage et, d'autre part, parce que l'on se rapproche dans ces conditions de la fermentation en milieu solide.

Selon une variante d'exécution on utilisera un mélange azote/dioxyde de carbone comme fluide d'espacement ; ce fluide sera particulièrement avantageux lorsque les microorganismes à cultiver seront des algues, car ce mélange favorisera l'activité de photosynthèse.

Quel que soit le fluide d'espacement utilisé, qui pourra se présenter sous forme d'un mélange de gaz, ledit fluide d'espacement devra :
- être non miscible avec le fluide porteur et le contenu des micro-bioréacteurs,
- ne pas interagir avec le microorganisme, c'est-à-dire que le microorganisme ne doit pas être capable de croître ou de se propager dans le fluide d'espacement,
- ne pas être-toxique, c'est-à-dire nuisible à la croissance du microorganisme.

Selon un mode préférentiel de réalisation du procédé selon l'invention, le diamètre des micro-bioréacteurs (5) sera inférieur à celui du tube capillaire (8) ; à titre encore plus préférentiel, le diamètre des micro-bioréacteurs (5) sera dans une fourchette comprise entre 80% et 85% du diamètre du tube capillaire (8). Une telle configuration sera particulièrement avantageuse lorsque les microorganismes à cultiver dans les micro-bioréacteurs (5) sont des champignons filamenteux. En dessous de la valeur de 80% il y a un risque de voir les bulles d'air successives constituant le fluide d'espacement (6) entrer en contact en dessous des micro-bioréacteurs (5), ce qui entraîne rapidement les fusions de bulles d'espacement (6) et des micro-bioréacteurs (5).

Le train de gouttes utilisé pour la croissance des champignons filamenteux sera avantageusement constitué selon le mode opératoire suivant.

Les spores de champignons filamenteux sont suspendues dans du milieu PGS (glucose 10g/L, peptone pancréatique 6g/L, MgSO₄ 7H₂O 0,5g/L, KH₂PO4 0,5g/L, FeSO₄ 7H₂O 0,5mg/L, pH ajusté à 5). Le liquide porteur est composé d'huile fluorée Novec 7500 (HFE). Le train est fabriqué au niveau d'une jonction en croix de diamètre intérieur 0,5mm connecté à un tube capillaire en FEP de 15m de long et de diamètre intérieur 0,75mm. Le milieu PGS et l'huile HFE sont injectés par des pousses-seringues à des débits respectifs de 5,0 et 3,5mL/h. L'air est injecté via une vanne solénoïde avec une pression de 0,5bar à travers un tube de 50cm de long et de diamètre intérieur de 0,2mm. Ce tube permet de créer une résistance hydrodynamique suffisante pour générer un train homogène. A chaque bord du train, des bulles d'air de 10cm de long sont injectées et permettent de confiner le train.

En outre, il a été observé dans le cas de croissance d'algues et de champignons filamenteux que les bulles d'air d'espacement (6) diminuaient au cours du temps du fait de l'activité biologique à l'intérieur des micro-bioréacteurs (5) (respiration et photosynthèse). Il en résulte que si les bulles d'espacement (6) sont trop petites au départ de la mise en oeuvre du procédé selon l'invention, il arrive un moment où certaines disparaissent entraînant la coalescence des micro-bioréacteurs (5) qu'elles séparaient. Avantageusement, pour obtenir un train stable sur plus de 90h, la taille d'une bulle d'espacement (6) doit être au moins dix fois plus grande que le diamètre interne du tube capillaire (8).

Les cinétiques étudiées étant longues, des effets de bords ont pu être observés. On a ainsi une diminution de la taille des micro-bioréacteurs (5) aux deux bords du train qui peut résulter en la coalescence du micro-bioréacteur (5) avec son voisin. Cet effet est aussi lié au fait que l'on utilise de l'air comme fluide d'espacement qui va favoriser l'évaporation. Pour s'affranchir de ce phénomène, un mode préférentiel de réalisation du procédé selon l'invention consiste à saturer en eau les réservoirs de fluide porteur permettant la recirculation du train.

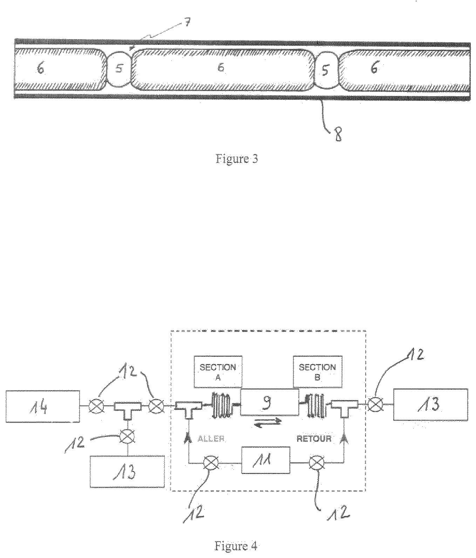

Un exemple va maintenant être donné de mise en ouvre du procédé selon l'invention.
1/ Préparation des solutions :
   - suspension de microorganismes dans le milieu de culture à concentration correspondant au taux d'occupation visé (nombre de cellules/-micro-bioréacteurs)
   - huile perfluorée saturée en eau additionnée de surfactant perfluoré (0,06%)
2/ Génération du train de gouttes avec :
   - fluide porteur : huile perfluorée HFE7500 + 0,06 % surfactant
   - fluide d'espacement : air comprimé
   - réactifs : suspensions de microorganismes
   Le ratio des débits/pressions des trois fluides est réglé pour obtenir :
   a) une taille de micro-bioréacteur (5) comprise entre 80 et 85% du diamètre interne du tube (8),
   b) une taille de bulle d'espacement (6) d'au moins trois fois le diamètre interne du tube (8).
   Les caractéristiques de taille figurant au a) et au b) ci-dessus apparaissent à la figure 3.
3/ Ajout de bouchons terminaux de fluide d'espacement sous forme d'une longue bulle (6) au début et à la fin du train de gouttes.
4/ Contrôle de la qualité du train de gouttes (80% < taille des micro-bioréacteurs < 85%), par analyse d'image.
5/ Suivi cinétique par mouvement d'aller-retour du train de gouttes devant le détecteur (9). 6/Analyse des données enregistrées pour chaque micro-bioréacteur.

A titre indicatif, le tableau ci-dessous reprend différents paramètres (fluide d'espacement, taille des micro-bioréacteurs ou des bulles du fluide d'espacement) et permet de voir quelle est la durée d'incubation maximale des microorganismes en fonction de ces paramètres.

| N° de l'essai | Taille du micro-bioréacteur (en % du diamètre interne du tube capillaire) | Fluide d'espacement | Taille des gouttes ou bulles du fluide d'espacement (en % du diamètre interne du tube) | Durée d'incubation maximale |
|---|---|---|---|---|
| 1 | 100% | Huile minérale | 200% | 48h |
| 2 | 100% | Huile minérale | 200% | 34h |
| 3 | 100% | Huile minérale | 200% | 28h |
| 4 | 100% | PDMS(100cSt) | 200% | 13h |
| 5 | 100% | PDMS(50cSt) | 200% | 20h |
| 6 | 100% | Squalène | 200% | 25h |
| 7 | 100% | Air | 1000% | 22h |
| 8 | 95% | Air | 1000% | 30h |
| 9 | 85% | Air | 1000% | 91h |
| 10 | 85% | Air | 600% | 60h |
| 11 | 90% | Air | 1200 % | 68h |
| 12 | 90% | Air | 1200% | 162h |
| 13 | 90% | Air | 1500% | 109h |

Dans le tableau ci-dessus, les microorganismes suivants ont été cultivés :
- *Aspergillus Oryzae* (champignon filamenteux) pour les essais 1-7 et 11 ;
- *Aspergillus Niger* (champignon filamenteux) pour les essais 8-10 ;
- *Chlamydomonas Reinhardti* (algue unicellulaire) pour les essais 12-13.

S'agissant des champignons filamenteux précités, les meilleurs résultats en termes de stabilité ont été obtenus dans les essais 9, 10 et 11.

Grâce aux améliorations apportées dans le cadre du procédé selon l'invention, il a pu être démontré qu'il était possible de suivre des cinétiques de croissance et d'activités biologiques dans des micro-bioréacteurs sur des durées pour le moment inaccessibles à l'homme de l'art.

Bien que les algues et les levures ne traversent pas la barrière huile porteuse/milieu de culture durant leur croissance, cette méthode de confinement résulte en une stabilité accrue du train de gouttes en réduisant l'interaction entre la goutte de milieu de culture et les parois du tube sachant que la diminution de la tension de surface liée aux métabolites sécrétés par les microorganismes durant leur croissance affecte la stabilité du train au cours du temps. Enfin, l'utilisation des bulles de gaz peut permettre de réguler les échanges gazeux en servant de réservoir d'oxygène (respiration des champignons filamenteux) ou de dioxyde de carbone (photosynthèse des algues).

Les micro-bioréacteurs (5) sont agencés en un train unidimensionnel pouvant varier de plusieurs centaines à plusieurs milliers d'échantillons. Chaque micro-bioréacteur (5) est identifié par son rang dans le train. L'intégrité du train de micro-bioréacteurs est ainsi essentielle pour assurer des réactions sur de longues durées. Les micro-bioréacteurs (5) sont mis en mouvement continuellement afin de préserver le film de lubrification et d'induire par recirculation une homogénéisation du micro-bioréacteur. En faisant passer le train devant un détecteur (9) tel qu'illustré à la figure 3 dans un sens puis dans l'autre, il est possible de suivre au cours du temps les réactions au sein de chaque micro-bioréacteur (5). Il est aussi possible de faire passer le train de micro-bioréacteurs (5) devant le détecteur toujours dans le même sens en assurant une boucle de recirculation, permettant de suivre au cours du temps les réactions au sein de chaque micro-bioréacteur.

Ce détecteur (9) est intégré dans un module d'incubation (10) comprenant notamment une pompe (11) et des vannes (12) (électrovannes ou vannes pneumatiques par exemple) permettant de faire circuler dans un sens puis dans l'autre le train de micro-bioréacteurs, ce dernier étant chargé dans la section A puis déplacé devant le détecteur (9) vers la section B. La présence de sorties (13) permet d'éliminer les micro-bioréacteurs (5) indésirables et de nettoyer le circuit une fois l'expérience terminée. Un module (14) complète le présent système, module dans lequel est constitué le train de gouttes (micro-bioréacteurs et bulles de fluide) conformément à la figure 1 avec les différents réservoirs contenant les phases (I), (II) et (III).

## Revendications

1. Procédé pour la culture de microorganismes par confinement dans des micro-bioréacteurs (5), du type comprenant un fluide porteur destiné à faire avancer dans un tube capillaire (8) un train de gouttes constitué de micro-bioréacteurs (5) dans lesquels a lieu la culture demicroorganismes susceptibles de modifier les interfaces desdites gouttes, où lesdits micro-bioréacteurs (5) sont séparés par un fluide d'espacement, **caractérisé en ce que** ledit fluide d'espacement est un gaz.

2. Procédé selon la revendication 1 **caractérisé en ce que** ledit gaz est l'air.

3. Procédé selon la revendication 1 **caractérisé en ce que** ledit gaz est un mélange d'azote et de dioxyde de carbone.

4. Procédé selon l'une quelconque des revendications 1 à 3 **caractérisé en ce que** le diamètre desdits micro-bioréacteurs (5) est inférieur à celui dudit tube capillaire (8).

5. Procédé selon la revendication 4 **caractérisé en ce que** ledit diamètre desdits micro-bioréacteurs (5) est compris entre 80% et 85% du diamètre dudit tube capillaire (8).

6. Procédé selon l'une quelconque des revendications 1 à 5 **caractérisé en ce que** ledit fluide d'espacement se présente sous forme d'une bulle (6) dont la taille est comprise dans une fourchette de deux à dix fois le diamètre dudit tube capillaire (8).

7. Procédé selon l'une quelconque des revendications 1 à 6 **caractérisé en ce que** ledit fluide porteur provient d'un réservoir saturé en eau.

8. Procédé selon l'une quelconque des revendications 1 à 6 **caractérisé en ce que** le microorganisme cultivé est un champignon filamenteux.

9. Procédé selon la revendication 8 **caractérisé en ce que** ledit champignon filamenteux est *Aspergillus Oryzae* ou *Aspergillus Niger.*

10. Procédé selon l'une quelconque des revendications 1 à 6 **caractérisé en ce que** le microorganisme cultivé est une algue planctonique.

11. Procédé selon la revendication 10 **caractérisé en ce que** ladite algue est *Chlamydomonas Reinhardti.*

## Patentansprüche

1. Verfahren zum Züchten von Mikroorganismen durch Einschließung in Mikrobioreaktoren (5) des Typs, der ein Trägerfluid umfasst, das dazu dient, in einem Kapillarrohr (8) einen Tropfenzug vorwärtszubringen, der aus Mikrobioreaktoren (5) besteht, in denen das Züchten von Mikroorganismen stattfindet, die die Schnittstellen der Tropfen verändern können, wo die Mikrobioreaktoren (5) durch ein Spaltfluid getrennt werden, **dadurch gekennzeichnet, dass** das Spaltfluid ein Gas ist.

2. Verfahren nach Anspruch 1, **dadurch gekennzeichnet, dass** das Gas Luft ist.

3. Verfahren nach Anspruch 1, **dadurch gekennzeichnet, dass** das Gas ein Gemisch aus Stickstoff und Kohlendioxid ist.

4. Verfahren nach einem der Ansprüche 1 bis 3, **dadurch gekennzeichnet, dass** der Durchmesser der Mikrobioreaktoren (5) kleiner als der Durchmesser des Kapillarrohrs (8) ist.

5. Verfahren nach Anspruch 4, **dadurch gekennzeichnet, dass** der Durchmesser der Mikrobioreaktoren (5) zwischen 80 % und 85 % des Durchmessers des Kapillarrohrs (8) beträgt.

6. Verfahren nach einem der Ansprüche 1 bis 5, **dadurch gekennzeichnet, dass** das Spaltfluid die Form einer Blase (6) aufweist, deren Größe innerhalb eines Bereichs von zwei- bis zehnmal des Durchmessers des Kapillarrohrs (8) liegt.

7. Verfahren nach einem der Ansprüche 1 bis 6, **dadurch gekennzeichnet, dass** das Trägerfluid aus einem mit Wasser gesättigten Reservoir stammt.

8. Verfahren nach einem der Ansprüche 1 bis 6, **dadurch gekennzeichnet, dass** der gezüchtete Mikroorganismus ein filamentöser Pilz ist.

9. Verfahren nach Anspruch 8 **dadurch gekennzeichnet, dass** der filamentöse Pilz *Aspergillus Oryzae* oder *Aspergillus Niger* ist.

10. Verfahren nach einem der Ansprüche 1 bis 6, **dadurch gekennzeichnet, dass** der gezüchtete Mikroorganismus eine Planktonalge ist.

11. Verfahren nach Anspruch 10, **dadurch gekennzeichnet, dass** die Alge *Chlamydomonas Reinhardti* ist.

## Claims

1. Method for cultivating micro-organisms by confinement in micro-bioreactors (5), of the type comprising a carrier fluid for moving a train of droplets in a capillary tube (8) comprising micro-bioreactors (5) in which the culture of micro-organisms takes place that can modify the interfaces of said droplets, wherein said micro-bioreactors (5) are separated by a spacing fluid, **characterised in that** said spacing fluid is a gas.

2. Method according to claim 1 **characterised in that** said gas is air.

3. Method according to claim 1 **characterised in that** said gas is a mixture of nitrogen and carbon dioxide.

4. Method according to any of claims 1 to 3 **characterised in that** the diameter of said micro-bioreactors (5) is less than that of said capillary tube (8).

5. Method according to claim 4 **characterised in that** said diameter of said micro-bioreactors (5) is between 80% and 85% of the diameter of said capillary tube (8).

6. Method according to any of claims 1 to 5 **characterised in that** said spacing fluid has the form of a bubble (6) of which the size is within a range of two to ten times the diameter of said capillary tube (8).

7. Method according to any of claims 1 to 6 **characterised in that** said carrier fluid comes from a reservoir that is saturated with water.

8. Method according to any of claims 1 to 6 **characterised in that** cultivated micro-organism is a thread-like fungi.

9. Method according to claim 8 **characterised in that** said thread-like fungi is *Aspergillus Oryzae* or *Aspergillus Niger.*

10. Method according to any of claims 1 to 6 **characterised in that** the cultivated micro-organism is a planktonic algae.

11. Method according to claim 10 **characterised in that** said algae is *Chlamydomonas Reinhardti.*
